# EUROPEAN PATENT APPLICATION

(11) **EP 2 260 859 A1**
(43) Date of publication of application: **15.12.2010**
(21) Application number: 09007772.8
(22) Date of filing: 12.06.2009
(51) Int. Cl.: A61K 38/12, C07K 7/64

(54) **Compounds acting as peptide gap junction modulators and their uses**

(71) Applicant: Zealand Pharma A/S, 2600 Glostrup (DK); The Research Foundation Of State University Of New York, Albany, NY 12207-2826 (US)
(72) Inventor: The designation of the inventor has not yet been filed
(74) Representative: Kiddle, Simon John

(57) **Abstract**

Compounds capable of modulating intracellular gap junctional communication, and their uses in the treatment of diseases associated with impaired gap junction intracellular communication (GJIC) are disclosed.

## Description

### FIELD OF THE INVENTION

The invention relates to compounds capable of modulating intracellular gap junctional communication, to pharmaceutical compositions comprising the compounds and their medical uses for the prevention and/or treatment of conditions associated with modulating gap junction communication.

### BACKGROUND OF THE INVENTION

There is increasing recognition that intercellular communication is essential for cellular homeostasis, proliferation and differentiation. Such communication is believed to be facilitated by gap junctions. These structures are thought to be a route for coupling cells and permitting "crosstalk". See generally, Sperelakis, N., (1989) Cell Interactions and Gap Junctions by N. Sperelakis, William C. Cole (Editor). Connexins are integral membrane proteins that oligomerize to form intercellular channels called gap junctions. The most abundant gap junction protein in a number of mammalian systems is connexin43 ("Cx43").

Gap junction channels are responsible for direct cell-to-cell communication. These channels are dynamic pores that are regulated in response to changes in the cellular environment and by protein interactions. In the heart, gap junction channels are a critical mechanism for the passage of electrical impulses (Lerner et al., "Accelerated Onset and Increased Incidence of ventricular arrhythmias induced by ischemia in Cx43-deficient mice," Circ. 101(5):547-552 (2000); Gutstein et al., "Conditional gene targeting of connexin43: exploring the consequences of gap junction remodeling in the heart," Cell Commun. Adhes. 8(4-6):345-348 (2001); Vaidya et al., "Null mutation of connexin43 causes slow propagation of ventricular activation in the late stages of mouse embryonic development," Circ. Res. 88(11):1196-1202 (2001)). Each channel is composed of two identical hexameric structures or connexons that dock across an extracellular space. The result is a permeable pore that is dynamically regulated. The individual subunit of the connexon is the molecule connexin. This molecule resides in the membrane, with its N-terminal ("NT"), cytoplasmic loop ("CL") and C-terminal ("CT") domains in the cytoplasmic space. In addition, there are four transmembrane domains and two extracellular domains that are involved in the docking to the opposing connexon. There are at least 20 different connexin isotypes in the mouse genome and 21 in the human genome (Willecke et al., "Structural and functional diversity of connexin genes in the mouse and human genome," Biol. Chem. 383(5):725-737 (2002)). The most abundant connexin isotype in the heart, brain and other tissues is the 43 KDa protein, Cx43.

Gap junctions allow the passage of ions and small molecules between cells and are regulated by a variety of chemical interactions between the connexin molecule and the microenvironment. As such, gap junctions act as active filters to control the passage of intercellular messages to modulate function.

Previous work has suggested that regulation of Cx43 channels results from the association of the CT domain, acting as a gating particle, and a separate region of the connexin molecule acting as a receptor for the gating particle (Duffy et al., "pH-Dependent Intramolecular Binding and Structure Involving Cx43 Cytoplasmic Domains," J. Biol. Chem. 277(39):36706-36714 (2002); Moreno et al., "Role of the Carboxyl Terminal of Connexin43 in Transjunctional Fast Voltage Gating," Circ. Res. 90(4):450-457 (2002), 92(1):e30 (2003) (erratum)). Additional studies have shown that this intra-molecular interaction can be modulated by other inter-molecular interactions in the microenvironment of the gap junction plaque (Morley et al., "Intramolecular Interactions Mediate pH Regulation of Connexin43 Channels," Biophys. J. 70(3):1294-1302 (1996)). Thus, the emerging picture of a Cx43 gap junction plaque is that of a macromolecular complex where proteins act in concert to modulate intercellular communication. At the center of these interactions is the CT domain, which acts as a substrate for a number of kinases.

Gap junction pharmacology is a nascent field. It has been recently shown that hexa-peptides such as AAP10 (a sequence initially derived from bovine atria), as well as its stable analogue ZP123 - now termed rotigaptide - together with a novel peptide, GAP134, have been found to modify gap junctional communication, and to show potential as antiarrhythmic agents (Müller et al, Actions of the antiarrhythmic peptide AAP10 on intercellular coupling, Naunyn Schmiedebergs Arch. Pharmacol. 1997 356(1):76-82; Kjolbye et al., Pharmacological modulation of gap junction function with the novel compound rotigaptide: a promising new principle for prevention of arrhythmias. Basic Clin. Pharmacol. Toxicol., 2007 101:215-30; Rossman et al., Effects of the gap junction modifier, GAP-134, on conduction and atrial fibrillation/flutter inducibility in dogs. AHA Scientific Sessions Abstract 1837, 2007 ; Axelsen et al., Increasing gap junctional coupling: a tool for dissecting the role of gap junctions. J. Membr. Biol. 2007;1:23-35.

This accumulated evidence supports the notion of gap junction modification as a suitable pharmacological target. However, further development of these molecules is limited by the fact that their precise molecular target remains undefined, clearly reducing their potential to work as platforms for target-specific drug design.

### SUMMARY OF THE INVENTION

Broadly, the present invention concerns work in which peptide and peptidomimetic compounds were designed based on knowledge on the molecular mechanisms of connexin43 regulation to identify a 34-amino acid peptide, dubbed RXP-E, able to bind the carboxyl terminal domain of Cx43 (Cx43CT), prevent cardiac gap junction closure and also prevent action potential propagation block. These previous studies allowed for a proof-of-concept as to the applicability of peptide-based pharmacology to Cx43, though the structure of the core active elements needed for pharmacological development remained undefined. The present inventors therefore used a combination of molecular modeling, surface plasmon resonance and patch clamp strategies to define, for the first time, a unique ensemble of pharmacophores that bind to the carboxyl terminal domain of Cx43 and prevent the closure of Cx43 channels, in particular compounds which are cyclic peptide or a cyclic peptidomimetic compounds.

Accordingly, in a first aspect, the present invention provides A compound which is a cyclic peptide or a cyclic peptidomimetic compound represented by Formula I

cyclo [Arg¹-Arg²-Pro³-Pro⁴-Tyr⁵-(Arg⁶)ₙ-X⁷]

wherein
n is 0 or 1;
X₇ is selected from Asn or a Glx group represented by the formula: 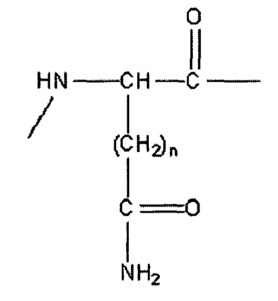 and n is 0, 2 or 6 and
X₇ is linked to Arg1 via a peptide bond to cyclise the peptide or peptidomimetic compound;
or a compound which differs from Formula I at one, two or three of the following positions whereby, if different from Formula I:
the residue at position Arg¹, Arg² and/or Arg⁶ is independently replaced by Lys, His or a Lysine mimetic group;
the residue at position Pro³ and/or Pro⁴ is independently replaced by a Proline mimetic selected from azetidine, hydroxyproline, morphoplino-3-carboxylic acid or an N-substituted amino acid such as Sarcosine, N-cyclohexylglycine or N-phenylglycine or 1-amino-cyclopentane carboxylic acid (Ac5c) or 1-amino-cyclohexane carboxylic acid (Ac6c);
the residue at position Tyr⁵ is replaced by Trp, naphthylalanine , Phe, Met, Val, Ile or Leu,
   or a retro analogue or a pharmaceutically acceptable salt thereof.

Preferred combinations of amino acid residue replacements are set out in the accompanying claims.

In a further aspect, the present invention provides a pharmaceutical composition comprising one or more of the compounds as defined herein and a pharmaceutical carrier. In a preferred embodiment, the composition is orally administrable. Preferably, the carrier is sterile, pyrogen-free and virus-free.

In a further aspect, the present invention provides a method for modulating gap junctional communication in a population of cells comprising contacting a population of cells with an effective amount of a compound as defined herein to modulate gap junctional communication between the cells. The method may be carried out *in vitro,* ex *vivo* or *in vivo.*

In a further aspect, the present invention provides a method of treating a patient having, or at risk of developing, a pathological condition involving impaired gap junctional communication comprising administering to the patient a therapeutically effective amount of a compound as defined herein. Typically, the patient will be a human being.

Examples of pathological conditions involving impaired gap junctional communication include cardiovascular disease, inflammation of airway epithelium, disorders of alveolar tissue, impaired hearing, endothelial lesions, diabetic retinopathy, diabetic neuropathy, ischemia of the central nervous system, ischemia of the spinal cord, dental tissue disorders, osteroporosis, kidney disease, failure of bone marrow transplantation, wounds, erectile dysfunction, urinary bladder incontinence, neuropathic pain, subchronic and chronic inflammation, cancer, transplantation failure, dermal disorders such as psoriasis and conditions caused by an excess of reactive oxygen species and/or free radicals and/or nitric oxide.

In a further aspect, the present invention provides a compound as defined herein for use in therapy. The therapy is generally a pathological conditions involving impaired gap junctional communication set out above.

In a further aspect, the present invention provide the use of a compound as defined herein in the manufacture of a medicament for the treatment of a pathological condition involving impaired gap junctional communication. The therapy is generally a pathological conditions involving impaired gap junctional communication set out above.

In embodiments in which the compounds contain amino acids residues, these may be D- or L-amino acids. In certain embodiments the use of D-amino acids is preferred to enhance stability. Preferably the amino acid residues within the peptides are α-amino acids. In other embodiments, they may be β-amino acids such as azetidin-3-carboxylic acid or γ-amino acids.

Embodiments of the present invention will now be described in more detail, by way of example and not limitation, with reference to the following figures.

### BRIEF DESCRIPTION OF THE FIGURES

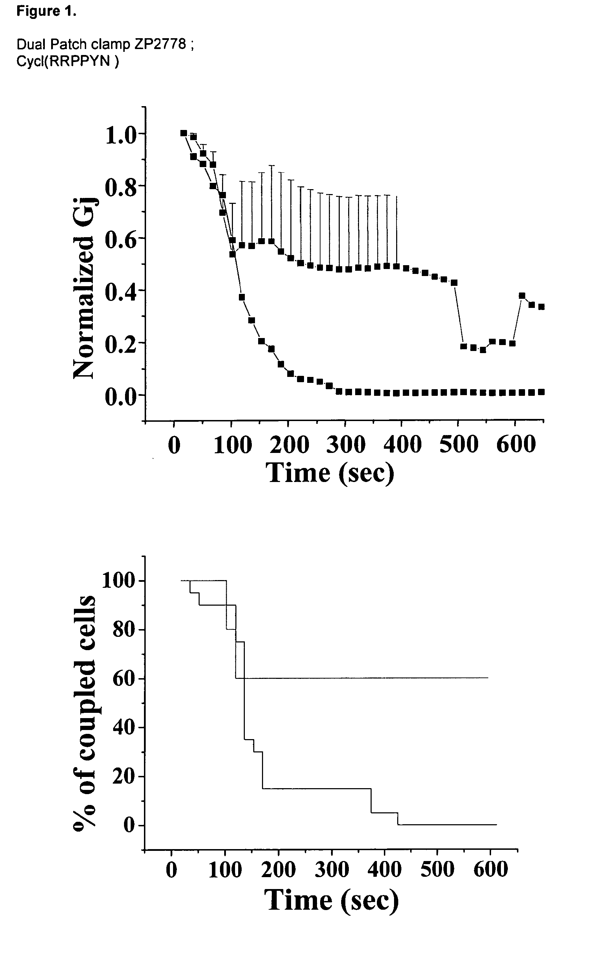

**Figure 1****.** SPR results. Recombinant rat Cx43CT was covalently bound to a carboxylmethyl dextran matrix and used as ligand. Peptides were presented to the bound ligand, and the amplitude of the response was subtracted from that obtained from a control chamber with no ligand. No change in angle of resonance was taken as an indication of absence of binding. The amplitude of the change in angle of resonance (expressed as "response units") is a function of the mass of the analyte and therefore, for comparison, response units were calibrated by the molecular weight of the tested compound. Change in angle of incidence of resonance (in "resonance units") was plotted against time after onset of cyclic peptide exposure. The different colors represent different concentrations applied of the peptide. Both peptides ZP2769 and ZP2771 shows significant binding to the Cx43CT, although ZP2769 seems to be less effective

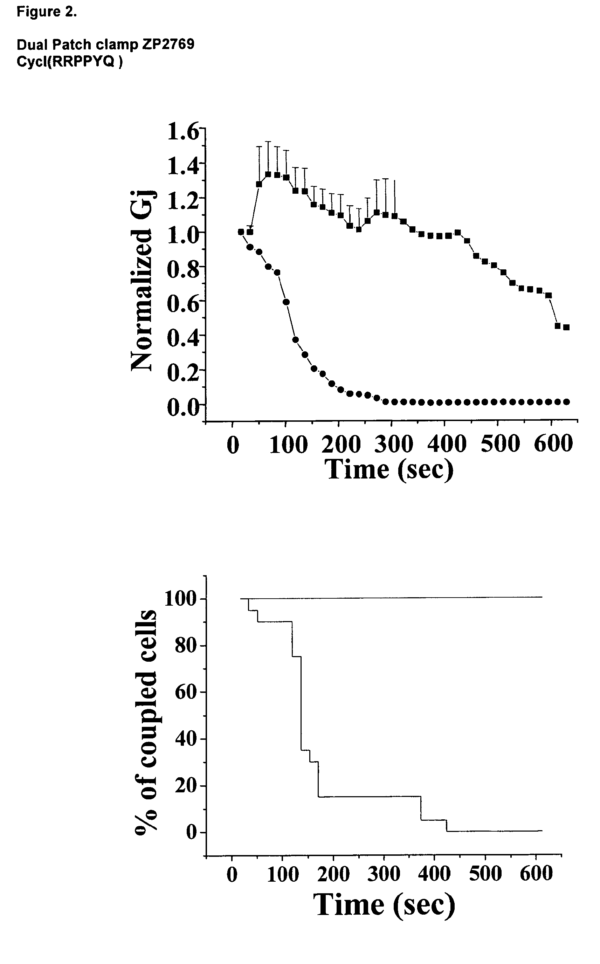

**Figure 2****.** Time course of octanol-induced uncoupling in human Cx43-expressing N2A cells . Cell pairs were recorded with patch pipettes that either contained ZP2778 (0.1 mM; red symbols), or only the internal pipette filling solution (black symbols). Experiments were conducted using the dual-cell patch clamp technique, and 1.5mM octanol perfusion was started at Time=0. An inclusion of ZP2778 in the patch pipette significantly (p<0.05) prevented the decrease of Gj compared to that of control.

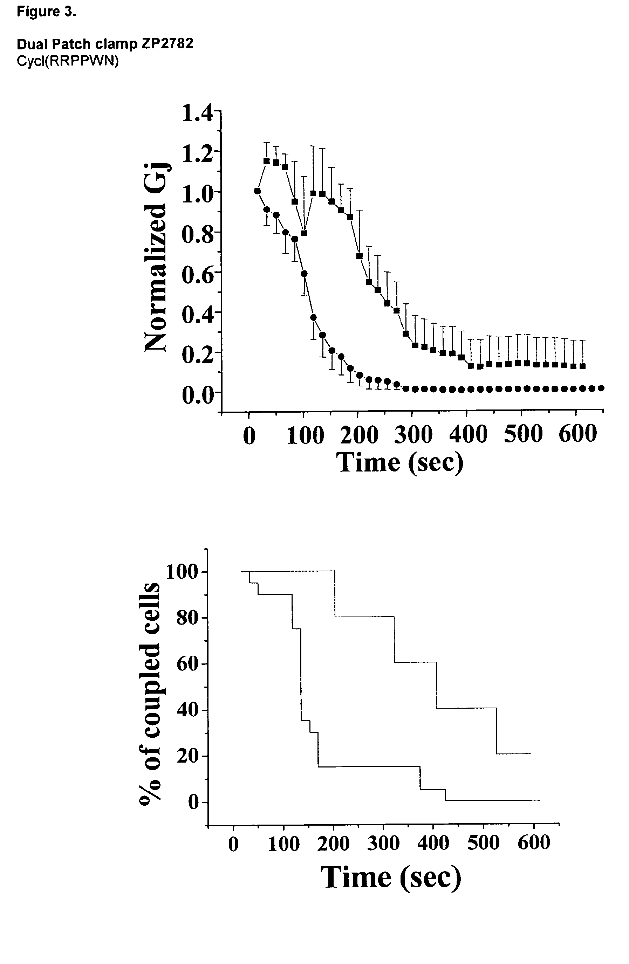

**Figure 3****.** Time course of octanol-induced uncoupling in human Cx43-expressing N2A cells. Cell pairs were recorded with patch pipettes that either contained ZP2769 (0.1 mM; red symbols), or only the internal pipette filling solution (black symbols). Experiments were conducted using the dual-cell patch clamp technique, and 1.5mM octanol perfusion was started at Time=0. Peptides ZP2769 and ZP2782 showed similar results with ZP2782 being less effective showing a decrease in overall Gj.

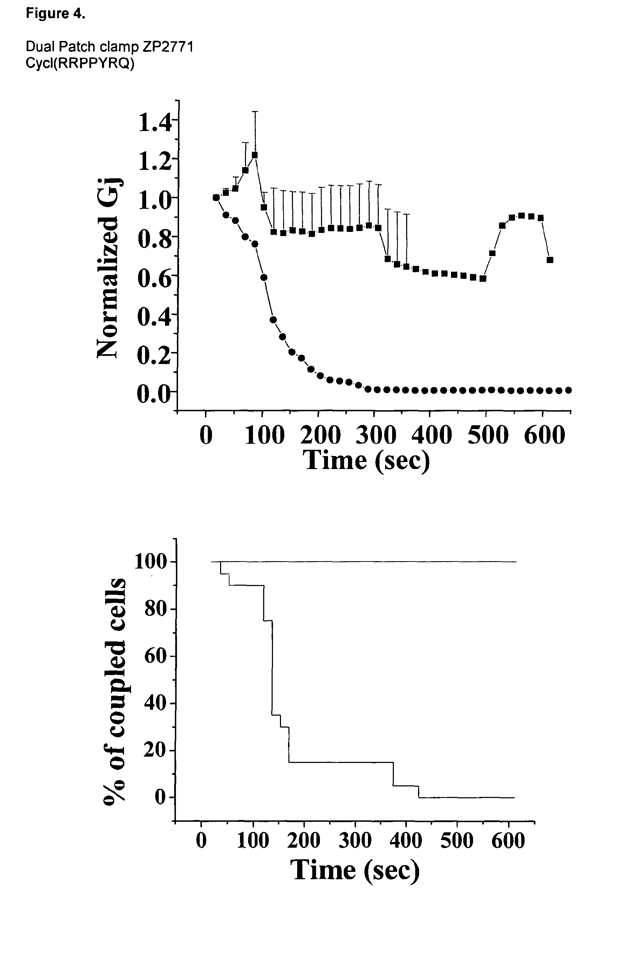

**Figure 4****.** Time course of octanol-induced uncoupling in human Cx43-expressing N2A cells. Cell pairs were recorded with patch pipettes that either contained ZP2782 (0.1 mM; red symbols), or only the internal pipette filling solution (black symbols). Experiments were conducted using the dual-cell patch clamp technique, and 1.5mM octanol perfusion was started at Time=0. Peptides ZP2769 and ZP2782 showed similar results with ZP2782 being less effective showing a decrease in overall Gj.

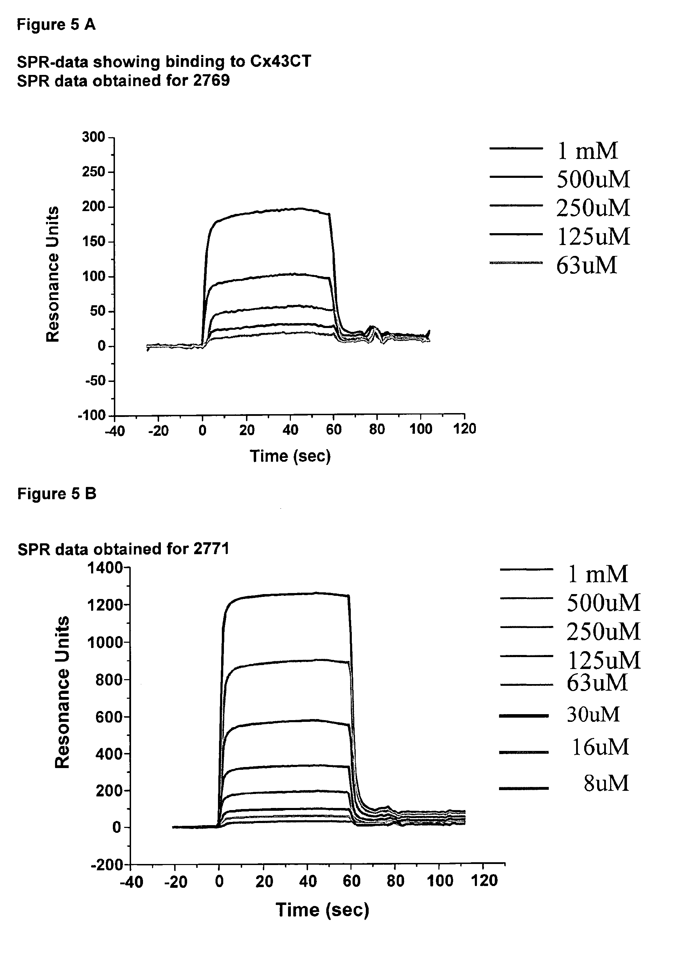

**Figure 5****.** Time course of octanol-induced uncoupling in human Cx43-expressing N2A cells. Cell pairs were recorded with patch pipettes that either contained ZP2771 (0.1 mM; red symbols), or only the internal pipette filling solution (black symbols). Experiments were conducted using the dual-cell patch clamp technique, and 1.5mM octanol perfusion was started at Time=0. Results obtained when ZP2771 was included in the intracellular filling solution prevented significantly (p<0.05) the decrease in Gj by octanol, and Gj remains at approximately 0.65 at the end of the experiment compared to control (Gj=0 after 10 minutes of octanol perfusion).

### DETAILED DESCRIPTION OF THE INVENTION

Before the preferred and exemplified compounds, compositions and uses are described, it is to be understood that this invention is not limited to the particular methodology, protocols and reagents described as such may, of course, vary. It is also to be understood that the terminology used herein is for the purpose of describing particular embodiments only, and is not intended to limit the scope of the present invention which will be limited only by the appended claims.

All publications mentioned herein are incorporated herein by reference to disclose and describe the methods and/or materials in connection with which the publications are cited. Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

As discussed above, the present invention generally relates to peptides that modulate gap junction intercellular communication (GJIC), and are preferably represented by Formula I as described above. The present invention also relates to methods for modulating gap junctional communication, methods of treating patients having, or at risk of developing, a pathological condition involving impaired gap junctional communication and pharmaceutical compositions comprising the inventive peptides. The present invention has a wide spectrum of useful applications including use in the treatment or prevention of pathologies associated with impaired GJIC.

### DEFINITIONS

Unless specified otherwise, the following definitions are provided for specific terms which are used in the following written description. Throughout the description and claims the three letter code for natural amino acids is used as well as generally accepted three letter codes for other α-amino acids, such as Sarcosin (Sar), α-Amino-iso-butanoic acid (Aib), Naphthylalanine (Nal) including 1-naphthylalanine (1Nal) and 2-naphthylalanine (2Nal), Phenylglycine (Phg), 2,4-Diaminobutanoic acid (Dab), 2,3-Diaminopropanoic acid (Dap), Diaminoacetic acid (Daa) and hydroxyproline (Hyp). Where nothing is specified Hyp represents 4-hydroxyproline or 3-hydroxyproline. The natural or essential amino acids are the amino acid constituents of proteins. The aromatic amino acids are Phe, Tyr, Trp, 1 Nal, 2Nal and His. Where the L or D form has not been specified it is to be understood that the amino acid in question can be either in the L or D form.

The term "retro analogue" is intended to mean a peptide whose sequence is the reverse of the named peptide.

The term "lysine mimetics" (Lm or LM) as used herein means the following compounds: 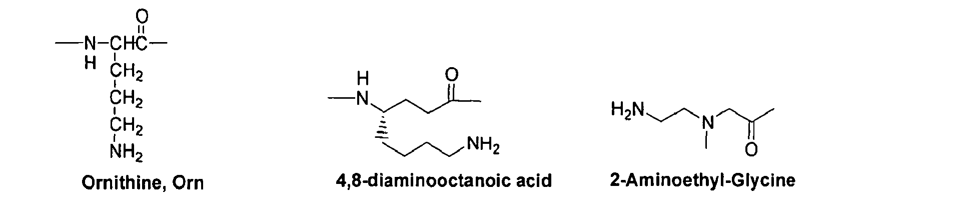 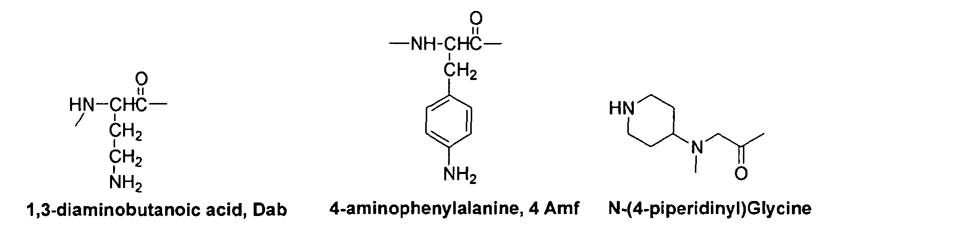 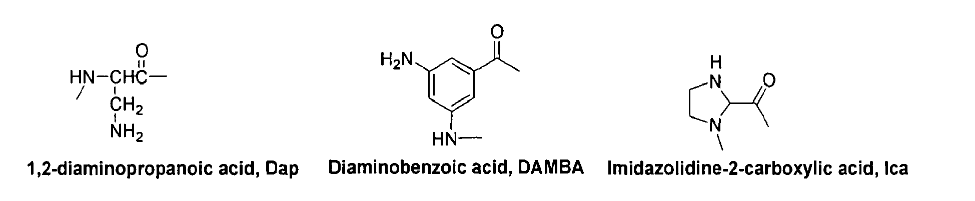 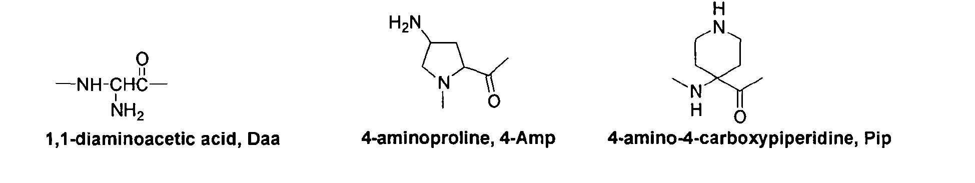 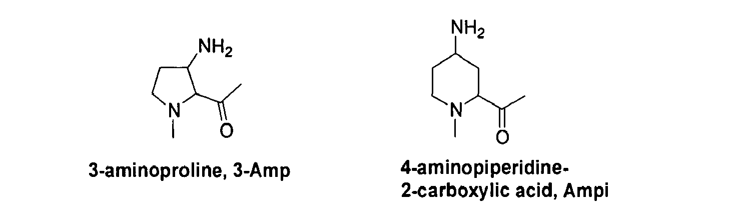 Examples of lysine mimetics having aliphatic cyclic amine groups and aryl amines include Damba, 4Amp, 4Ampi, Ica, Pip, and 4AmF, are included above.

Amp: 4-aminoproline (2S,4S; 2S,4R; 2R,4S; 2R,4R)(4-Amp) or 3-aminoproline, (2S,3S; 2S,3R; 2R,3S; 2R,3R) (3-Amp) (4 isomers of each).

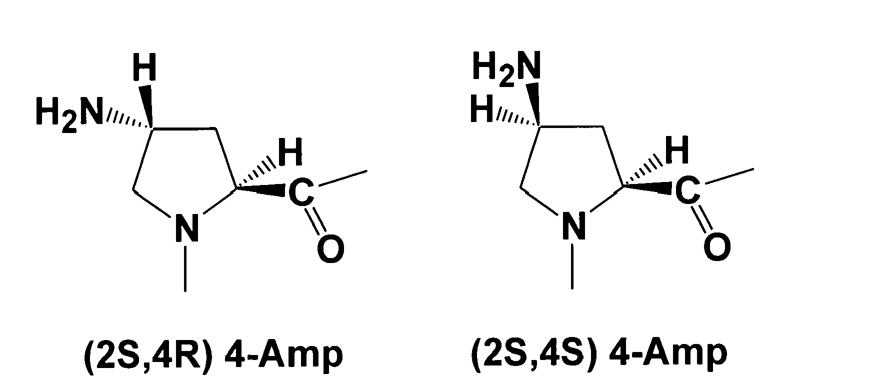

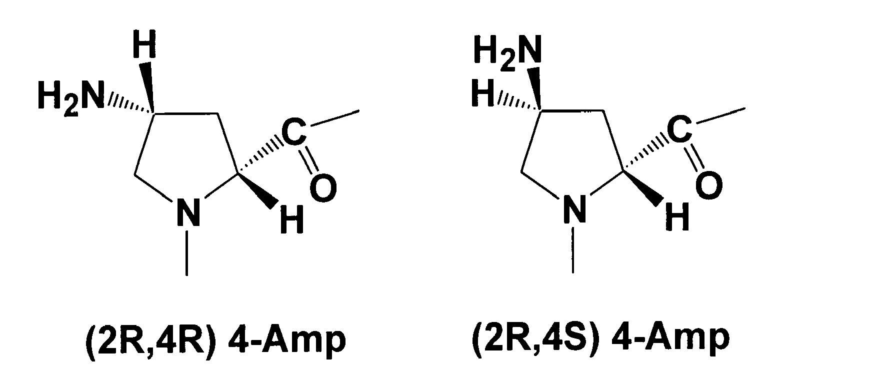

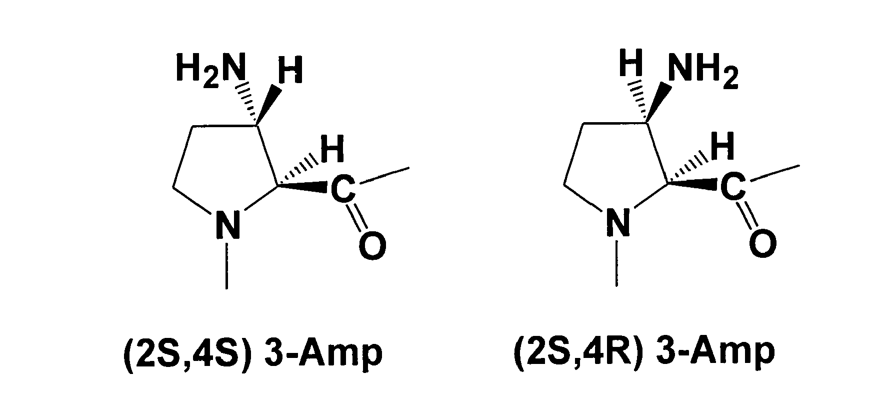

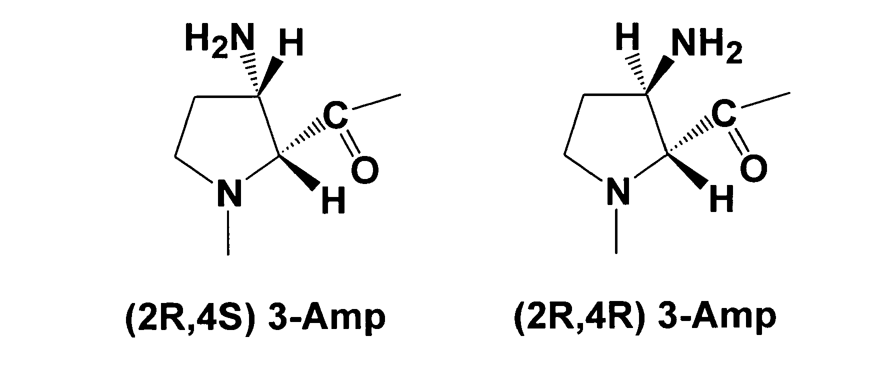

The term "proline mimetics" as used herein means the following compounds:

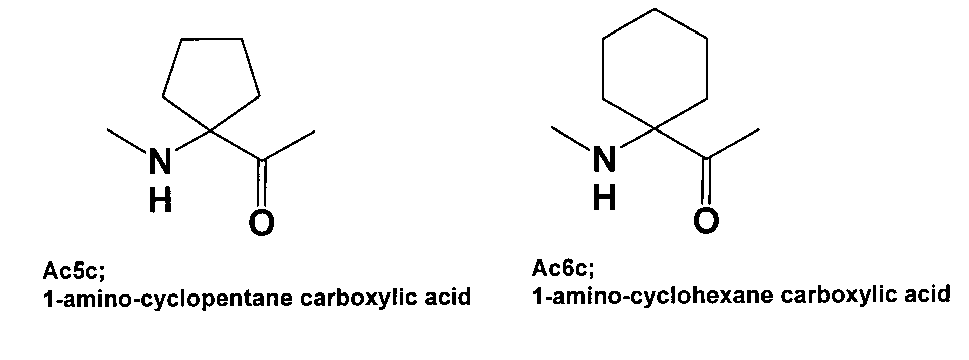

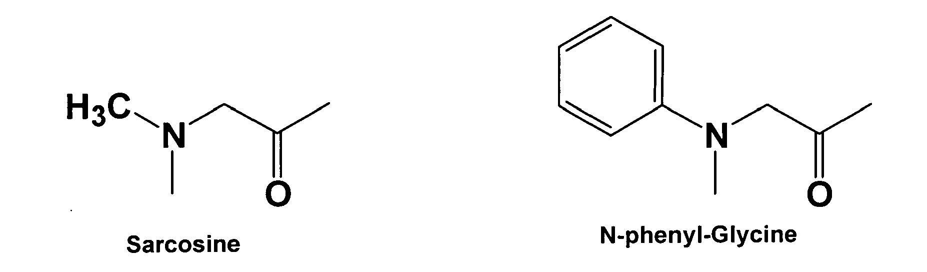

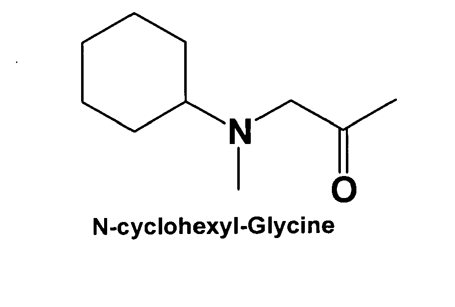

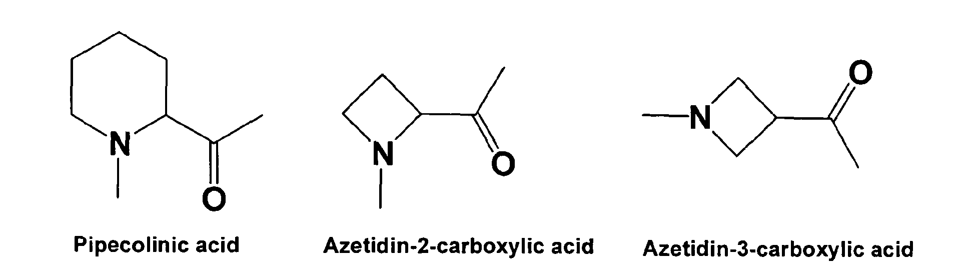

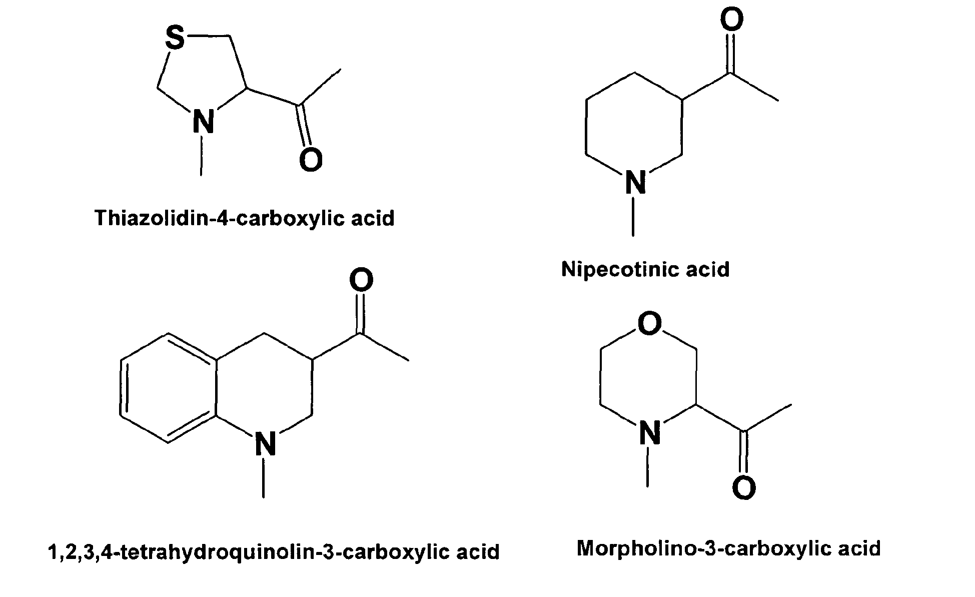

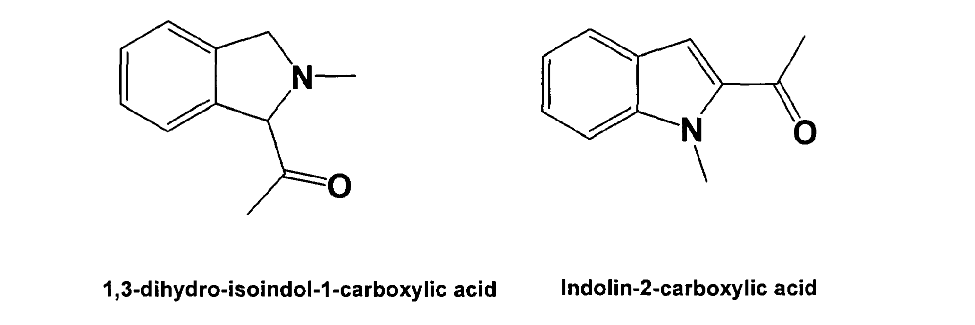

The terms "intercellular communication modulator", "gap junction facilitator", "compound that facilitates gap junction communication" and "gap junction opener", etc., all refer to a compound that facilitates, or maintains, or normalizes (e.g. either by inhibiting of enhancing), GJIC, irrespective of the particular mechanism behind this action. More specifically, the term "gap junction opener" may refer to a substance which normalizes (i.e., increases) the exchange of molecules that are able to pass through gap junctions between extracellular and intracellular spaces and/or which can normalize increase GJIC.

The term "agonist" refers to a compound (here, cyclic peptides or peptidomimetics) that can interact with a tissue, cell or cell fraction which is the target of for example, but not limited to, an AAP, AAP10, HP5 peptide, or functional analog thereof, to cause substantially, or at least substantially the same physiological responses in the tissue, cell or cell fractions the AAP, AAP10, HP5 peptide, or functional analog thereof. By way of example, the physiological response is one or more of: contraction, relaxation, secretion, and enzyme activation. Preferably, the compound binds to the tissue, cell or cell fraction. By way of example, the compound may bind to a receptor on the tissue, cell, or cell fraction, which binds to AAP, AAP10, HP5, or a functional analog thereof. However, the present "agonist" may interact with a tissue, cell or cell fraction, which is the target of any given compound, causing the same, or at least substantially the same physiological response.

The term "antagonist" refers to a compound (here, cyclic peptides or peptidomimetics) which inhibits or antagonizes one or more physiological responses observed in a tissue, cell or cell fraction after contacting the tissue, cell, or cell fraction with any given compound, such as AAP, AAP10, HP5 peptide, or a functional analog thereof. By way of example, the physiological response is one or more of: contraction, relaxation, secretion, and enzyme activation. Preferably, the compound binds to the tissue, cell or cell fraction. By way of example, the compound may bind to a receptor on the tissue, cell, or cell fraction which binds to AAP, AAP10, HP5, or a functional analog thereof and/or which inhibits binding of one or more of AAP, AAP10, HP5, a functional analog thereof to the receptor.

Examples of compounds according to the present invention are given below. The following are preferred cyclic peptides according to Formula I of the present invention. The corresponding retro forms according to Formula II are also examples of compounds according to the present invention. The term "retro analogue" includes a peptide or peptidomimetic having a sequence that is the reverse of a reference or parent peptide. Accordingly, Formula II may be written in full as:

cyclo [X⁷-(Arg⁶)ₙ-Tyr⁵-pro⁴-Pro³-Arg²-Arg¹]

wherein the substitituents defined by the formula have the identity as the correspoding substituents of Formula I.
Cyclo(Arg-Arg-Pro-Pro-Tyr-Gln),
Cyclo(Arg-Arg-Pro-Pro-Tyr-Arg-Gln),
Cyclo(Arg-Arg-Pro-Pro-Tyr-Asn),
Cyclo(Arg-Arg-Pro-Pro-Trp-Asn,
or a retro analogue or a pharmaceutically acceptable salt thereof.

Each amino acid is independently an L-amino acid or a D-amino acid. Pharmaceutically acceptable salts of the above preferred compounds are also within the scope of the present invention.

### Properties of the Compounds

The activity of compounds of the present invention may be tested for binding to the carboxyl terminal domain of Cx43 using Surface Plasmon Resonance ("SPR"). SPR is a method that was used to identify the original RXPE sequence (Shibayama et al., "Identification of a Novel Peptide that Interferes with the Chemical Regulation of Connexin-43," Circ. Res. 98:1365-72 (2006)). The concentration of the compound relative to the amplitude of the response in arbitrary units is measured.

It is preferred that the compounds of the present invention facilitate and/or maintain or inhibit the intercellular communication mediated by gap junctions. The present invention also relates to the preparation and use of pharmaceutical compositions for the treatment of pathologies associated with impaired intercellular gap junctional communication and methods for using these compositions.

Preferred compounds of the present invention show good function as a modulator of gap junctional communication (e.g., as agonists or antagonists) and may therefore have uses as antiarrhythmic drugs.

### MEDICAL USES

The present invention relates to compounds that are capable of modulating gap junction intercellular communication (GJIC). This may include one or more of the following functions: the ability to decrease cellular uncoupling, to normalize dispersion of action potential duration, and to normalize conduction velocity, the ability to control of the cellular quantity of gap junctions normalizing (up-regulating or down-regulating as needed) the expression of connexins; to normalize degradation of gap junctions (inhibit or enhance), to normalize cellular trafficking of connexins to the plasma membrane (increase or decrease); to facilitate assembly of connexins into functional gap junctions; to normalize opening of existing gap junctions, e.g., inducing or enhancing opening when they have been closed or gated by inhibitors (e.g., such as by mediating or enhancing hyperphosphorylation of the cytoplasmic carboxy terminal domain of one or more connexins (e.g., such as Cx43)) or closing these when they are aberrantly opened (e.g., as in Charcot-Marie-Tooth disease); and the like.

Particular assays useful for identifying and optionally quantifying the activity of the compounds are described below. The assays are non-limiting and are merely serving the purpose of illustrating a variety of assays in which the present compounds may be tested for their gap junction modulating abilities. It is to be understood that the assays are not mutually exclusive, i.e. a compound may show activity in one particular assay, but show a different, or no, activity in another particular assay.

### Arrhythmia

In one preferred aspect, the invention provides a compounds having antiarrhythmic properties that may be useful for the treatment of arrhythmias and thrombotic complications arising during cardiovascular disorders, such as acute ischemic heart disease (e.g., stable angina pectoris, unstable angina pectoris, acute myocardial infarction), congestive heart failure (e.g., systolic, diastolic, high-output, low-output, right or left sided heart failure), congenital heart diseases, cor pulmonale, cardiomyopathies, myocarditis, hypertensive heart disease, during coronary revascularization, and the like.

In specific embodiments, the compounds may be used to treat and/or prevent bradyarrhythmias (e.g., due to disease in sinus node, AV node, bundle of His, right or left bundle branch), and tachyarrhythmias associated with reentry (e.g., atrial premature complexes, AV junctional complexes, ventricular premature complexes, atrial fibrillation, atrial flutter, paroxymal supraventricular tachycardia, sinus node reentrant tachycardia, AV nodal reentrant tachycardia, and non-sustained ventricular tachycardia) either alone or in combination with other antiarrhythmic compounds, such as class I agents (e.g., lidocaine), class II agents (e.g., metoprolol or propranolol), class III agents (e.g., amiodarone or sotalol) or class IV agents (e.g., verapamil).

Additionally or alternatively, compounds may be used to treat one or more of: a reentry arrhythmia; ventricular reentry (e.g., such as arises during acute myocardial infarction, chronic myocardial infarction, stable angina pectoris and unstable angina pectoris); infectious or autonomic cardiomyopathy; atrial fibrillation;repolarization alternans; monomorphic ventricular tachycardia; T-wave alternans; bradyarrhythmias; and generally, reduced contractility of cardiac tissue, thrombosis and the like.

The antiarrhythmic properties of the compounds of the present invention may be tested in an assay measuring the increase in time to an AV block in a mouse after infusion of CaCl₂, in what is referred to herein as a "standard calcium-induced arrhythmia assay".

Compounds of the present invention may additionally show decreases in the incidence of reentry arrhythmias or in the size of an infarct zone observed in what is referred to herein as a "standard ventricular reentry assay".

### Diabetes

There is an understanding that GJIC is important in treatment of diabetes, for example in the treatment of diabetes type 1 or type 2, obesity, eating disorders and insulin resistance syndrome. The compounds of the present invention may improve blood glucose tolerance in diabetic mammals as determined by assays known in the art.

In specific embodiments, the compounds of the present invention may be used to stimulate insulin release, lower blood glucose level, reduce gastric motility, delay gastric emptying, inhibit food uptake, e.g. by suppression of appetite, or lower the plasma lipid level in a vertebrate or a mammal. The compounds may also be used generally in the treatment of diabetes mellitus associated with a risk for hyperglycemia, i.e. where insulin sensitivity is decreased with stress, myocardia infection, stroke and infections, or in cases of insulin resistance during pregnancy. The compounds may also be used in the treatment of other types of diabetes, such as cases where diabetes may be secondary to other endocrine diseases such as acromegaly, Cushing's syndrome, pheochromocytoma, glucagonoma, somatostatinoma, primary aldosteronism, or secondary to adminstration of certain hormones causing hyperglycemia, or secondary to certain drugs (antihypertensive drugs, thiazide diuretics, preparations containing estrogen, psychoactive drugs, sympathomimetic agents. Furthermore, the compounds may be used generally in the treatment of diseases and conditions associated with a risk for hypoglycemia, i.e. where endogenous glucose production is decreased, as following alcohol ingestion, or in cases where the sensitivity to insulin is increased in patients with hypopituitarism or primary adrenocortical insufficiency, or where insulin clearance is decreased as with progressive renal insufficiency.

### Osteoporosis

The compounds disclosed herein may be used to prevent and/or treat osteoporosis or other pathologies affecting bone formation, growth or maintenance. Compounds which are able to normalize the attenuated GJIC between human osteoblast during hypoxia are particularly suitable for the treatment of bone diseases with impaired bone formation relative to bone resorption. Suitable compounds for use in such methods can be selected in assays for increased alkaline phosphatase (ALP) activity in osteoblasts, which provides a means to monitor cell viability and growth as a consequence of proper maintenance of GJIC. In one aspect, human osteoblasts are stimulated with different concentrations of peptides from 1 x 10⁻³ to 1 x 10⁻⁶, and compared to untreated controls. Under normal culture conditions,compounds preferably increase ALP activity. Even more preferably, the compounds stimulate ALP activity during hypoxic conditions at concentrations ranging from 10⁻¹¹ to 10⁻⁸ mol/I. This can thus be used to optimize compounds selection and design for the treatment and/or prevention of bone diseases associated with poor vascularization, hypoxia and ischemia in bone tissue.

The compounds of the present invention may also be used for the prevention and/or treatment of joint diseases that involves impaired cell-to-cell coupling. This may include their use for the prevention and/or treatment of joint diseases that involve metabolic stress, for example forms of arthritis associated with decreased vascularization or healing of fractured cartilage tissue.

The function of the compounds in modulating GJIC in bone formation may be assessed using an assay in which the compound are tested to determine whether they increase osteoblast activity in a "standard osteoblast activity assay" which measures either calcium wave formation and/or alkaline phosphatase activity of osteoblast cells in the presence of the compounds. Preferably, such compounds increased calcium wave activity, manifested as an increase in the number of cells involved in a wave (as determined by measuring levels of intracellular Ca²⁺ using a calcium sensitive fluorescent dye, such as fura-2 and counting the number of cells which fluoresce). Alkaline phosphatase activity also can be used to provide a measure of osteoblast activity using standard colorimetric assays.

### Wounds

The compounds disclosed herein may be used to treat wounds and, in particular, to accelerate wound healing. Wound healing involves the interactions of many cell types, and intercellular communication mediated by gap.junctions is considered to play an important role in the coordination of cellular metabolism during the growth and development of cells involved in tissue repair and regeneration (K. M. Abdullah, et al. (1999) Endocrine, 10 35-41; M. Saitoh, et al. (1997) Carcinogenesis, 18: 1319-1328; J. A. Goliger, et al. (1995) Mol.Biol.Cell, 6 1491-1501). The compounds may be administered to the site of a wound by topical administration using carriers well known in the art (e.g., ointments, creams, etc.) or may administered systemically, e.g., for treating wounds of internal tissues, such as in the treatment of chronic gastric ulcer lesions. Additional functions in which endothelial gap-junctional intercellular communication has been implicated are the migratory behavior of endothelial cells after injury, angiogenesis, endothelial growth and senescence, and the coordination of vasomotor responses (G. J. Christ, et al. (2000) Braz. J Med Biol.Res., 33: 423-429). Therefore,the compounds diclosed herein may enhance conducted vascular responses and to improve blood supply during conditions with increased metabolic demand (e.g., physical exercise, tachycardia), and during ischemia.

Gap junctions are also believed to provide the molecular link for coordinated long-range signaling among individual members of the glial compartment. Likewise, astrocytes are ideally suited for metabolic support of neurons since they are functionally polarized with one extremity touching the vascular bed and the other pole approximates neuronal parenchyma (R. Dermietzel (1998) Brain Res. Brain Res. Rev., 26: 176-183). Thus, the compounds of the present invention may be administered to a patient in need to prevent ischemic damage in the brain by increasing the metabolic support between glia cells and neurons. Such patients may include patients with organic psychoses, which may present with signs such as depression, anxiety, learning and memory deficit, phobias, and hallucinations or patients who have suffered a traumatic brain injury. For this application, preferably the compounds are selected or formulated so as to be available to the central nervous system (i.e., provided with or conjugated with carriers which facilitate transport across the blood-brain barrier).

Compounds according to the invention may also be used to accelerate repair after nerve injury or during grafting of immature cells (progenitor cells) into brain tissue, e.g., such as in patients with neurotrauma, brain ischemia and chronic neurodegenerative diseases, such as Parkinson's disease or Huntington's disease (H. Aldskogius, et al. (1998) Prog. Neurobiol, 55: 1-26).

In addition, the compounds of the present invention may, due to the effect on the intercellular gap junction channels, be used to treat and/or prevent cataract (D. Mackay, et al. (1999) Am J Hum.Genet, 64 1357-1364) treat and/or prevent vascularization of the cornea in disease states with poor nutrition of the cornea and increase the healing of corneal lesions (S. G. Spanakis, et al. (1998) Invest Ophthalmol.Vis.Sci., 39: 1320-1328) and/or prevent hypertension.

In a further aspect, the compounds disclosed herein may be used to treat wounds and, in particular, to accelerate wound healing. Wound healing involves the interactions of many cell types, and intercellular communication mediated by gap junctions is considered to play an important role in the coordination of cellular metabolism during the growth and development of cells involved in tissue repair and regeneration (K. M. Abdullah, et al. (1999) Endocrine, 10 35-41; M. Saitoh, et al. (1997) Carcinogenesis, 18: 1319-1328; J. A. Goliger, et al. (1995) Mol.Biol.Cell, 6 1491-1501). Compounds may be administered to the site of a wound by topical administration using carriers well known in the art (e.g., ointments, creams, etc.) or may administered systemically, e.g., for treating wounds of internal tissues, such as in the treatment of chronic gastric ulcer lesions.

Additional functions in whch endothelial gap-junctional intercellular communication has been implicated are the migratory behavior of endothelial cells after injury, angiogenesis, endothelial growth and senescence, and the coordination of vasomotor responses (G. J. Christ, et al. (2000) Braz. J Med Biol.Res., 33: 423-429). Therefore, ithe compounds of the present invention may be used to enhance conducted vascular responses and to improve blood supply during conditions with increased metabolic demand (e.g., physical exercise, tachycardia), and during ischemia.

Gap junctions are also believed to provide the molecular link for coordinated long-range signaling among individual members of the glial compartment. Likewise, astrocytes are ideally suited for metabolic support of neurons since they are functionally polarized with one extremity touching the vascular bed and the other pole approximates neuronal parenchyma (R. Dermietzel (1998) Brain Res. Brain Res. Rev., 26: 176-183). Therefore, in one preferred embodiment, compounds of the present invention are administered to a patient in need to prevent ischemic damage in the brain by increasing the metabolic support between glia cells and neurons. Such patients may include patients with organic psychoses, which may present with signs such as depression, anxiety, learning and memory deficit, phobias, and hallucinations or patients who have suffered a traumatic brain injury. Preferably, such compounds are selected or formulated so as to be available to the central nervous system (i.e., provided with or conjugated with carriers which facilitate transport across the blood-brain barrier).

Compounds according to the invention may also be used to accelerate repair after nerve injury or during grafting of immature cells (progenitor cells) into brain tissue, e.g., such as in patients with neurotrauma, brain ischemia and chronic neurodegenerative diseases, such as Parkinson's disease or Huntington's disease (H. Aldskogius, et al. (1998) Prog. Neurobiol, 55: 1-26).

Ina further embodiment, compounds of the present invention may, due to the effect on the intercellular gap junction channels, be used to treat and/or prevent cataract (D. Mackay, et al. (1999) Am J Hum.Genet, 64 1357-1364) treat and/or prevent vascularization of the cornea in disease states with poor nutrition of the cornea and increase the healing of corneal lesions (S. G. Spanakis, et al. (1998) Invest Ophthalmol.Vis.Sci., 39: 1320-1328) and/or prevent hypertension.

### Psoriasis

In a further aspect, the compounds disclosed herein may be used to treat psoriasis. Psoriasis is a complex multifactorial condition resulting in characteristic psoriatic plaques and lesions. It is characterised by disordered epidermal cell proliferation and differentiation. There is a strong association with susceptibility to the epidermal differentiation complex - a loci of up to 50 genes with roles in epidermal differentiation, suggesting that impaired barrier function, or recovery seem to contribute to the progression of the psoriatic state (Capon et al.,2001; Djalilian et al 2006).

Central to maintenance of healthy skin is a tight and highly ordered cell to cell adhesion network including adherens, desmosomes and gap junction intercellular communication channels - the epidermal junctional nexus (Laird, 2006; Prochnow and Dermietzel, 2008). These intercellular junctions play crucial roles in epidermal proliferation, migration and differentiation and are all altered to differing degrees during psoriasis. Expression of connexins (Cx), the constituent proteins of gap junctions, is spatially and temporally regulated within the epidermis with Cx43 in basal and spinous layers normally being replaced by Cx26 in the upper granular layers (Di et al., 2001). In psoriatic plaques, however, Cx26 is greatly increased throughout the epidermis in a pattern similar to that in other hyperproliferative, stratified epithelia. In addition to the spatial redistribution of Cxs found in psoriasis there is also an associated down regulation of E-cadherin in the basal cell and upper spinous layers compared to normal epidermis (Chung et al., 2005). Thus, the hyperproliferation and altered differentiation status of psoriatic keratinocytes is associated with extensive remodelling of both gap- junctions and adherens junctions. The carboxyl terminal tails of Cx43, and cadherins are subject to post translational modification, particularly phosphorylation and interact with a variety of proteins including ZO-1 and β-catenin that serve as a link between the membrane incorporated protein and the cytoskeletal network (Laird, 2006; Prochnow and Dermietzel, 2008). The compounds disclosed herein are known to maintain and regenerate Cx43 GJIC under stress conditions and may therefore be used for the treatment of psoriasis inhibiting the down regulation of Cx43 and replacement with Cx26.

### Cancer

In a further aspect, the compounds disclosed herein may be used to treat cancer. Carcinogenesis is characterized by the progressive impairment of growth control mechanisms in which growth factors, oncogenes and tumor suppressor genes are involved. A general theme in carcinogenesis and tumorigenesis is the down regulation of the GJIC. Permeability of gap junctions in tumor cells using the dye-transfer assay is typically lower than GJIC in surrounding tissue. Further, the gating of gap junctions is known to be effected by tumor promoters, which decrease GJIC. This means that compounds of the present invention are used as medicaments for the treatment of cancer, alone, or in conjunction with traditional anti-cancer therapies.

The suitability of the compounds for treating cancer may, for example, be assessed using an assay in which the decrease of GJIC inhibition mediated by tumor promoters, such as DTT, is determined. In this "standard tumor promoter assay", preferably, the compounds show decreases in GJIC inhibition, which are at least 50%, preferably 70%, and more preferably 100% or greater, than decreases observed for AAP.

### Pharmaceutical Compositions

The compounds of the present invention may be formulated as pharmaceutical compositions comprising one or more of the compounds disclosed herein, in combination with a pharmaceutically acceptable carrier and/or diluent. Such compositions are preferably in a form adapted for oral administration. Formulations for oral administration may be prepared in a manner well-known to the person skilled in the art, e.g., as generally described in "Remington's Pharmaceutical Sciences", 17. Ed. Alfonso R. Gennaro (Ed.), Mark Publishing Company, Easton, PA, U.S.A., 1985 and more recent editions and in the monographs in the "Drugs and the Pharmaceutical Sciences" series, Marcel Dekker. By way of example, the compositions are in the form of tablets, capsules, granules, pellets, troches, lozenges, and the like. Suitable enteric formulations are described, e.g., in U.S. Patent No. 5,350,741.

The pharmaceutical carrier or diluent employed may be a conventional solid or liquid carrier. Examples of solid carriers are lactose, terra alba, sucrose, cyclodextrin, talc, gelatin, agar, pectin, acacia, magnesium stearate, stearic acid or lower alkyl ethers of cellulose. Examples of liquid carriers are syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax.

If a solid carrier is used for oral administration, the preparation may be tabletted, placed in a hard gelatin capsule in powder or pellet form, or it can be in the form of a troche or lozenge. The amount of solid carrier will vary widely but will usually be from about 25 mg to about 1 g. If a liquid carrier is used, the preparation may be in the form of a syrup or liquid suitable for oral ingestion.

It will be appreciated that the actual preferred amounts of compounds used in a given therapy will vary according to e.g. the specific compound being utilized, the particular composition formulated, the mode of administration and characteristics of the subject, e.g. the species, sex, weight, general health and age of the subject. Optimal administration rates for a given protocol of administration can be readily ascertained by those skilled in the art using conventional dosage determination tests conducted with regard to the foregoing guidelines. Suitable dose ranges may include from about 1 mg/kg to about 100mg/kg of body weight per day.

The compounds of the present invention are suitably administered in a protonated and watersoluble form, e.g., as a pharmaceutically acceptable salt, typically an acid addition salt such as an inorganic acid addition salt, e.g., a hydrochloride, sulfate, or phosphate salt, or as an organic acid addition salt such as an acetate, maleate, fumarate, tartrate, or citrate salt. Pharmaceutically acceptable salts of therapeutic compounds of the invention also can include metal salts, particularly alkali metal salts such as a sodium salt or potassium salt; alkaline earth metal salts such as a magnesium or calcium salt; ammonium salts such an ammonium or tetramethyl ammonium salt; or an amino acid addition salts such as a lysine, glycine, or phenylalanine salt. The compounds of the invention may also be administered topically to treat peripheral vascular diseases and as such may be formulated as a cream or ointment.

The compounds may also be formulated in compositions such as sterile solutions or suspensions for non-oral administration. This includes administration parenterally, that is by intravenous, intramuscular, subcutaneous, intranasal, intrarectal, intravaginal or intrapritoneal administration.

The compounds and compositions disclosed herein may be used to treat conditions or pathologies associated with impaired (abnormal decreases or increases in) gap junctional communication. In accordance with normal clinical practice, the compounds or compositions are administered to a patient in a therapeutically effective amount. As used herein, "a therapeutically effective amount" is one which reduces symptoms of a given condition or pathology, and preferably which normalizes physiological responses in a patient with the condition or pathology. Reduction of symptoms or normalization of physiological responses can be determined using methods routine in the art and may vary with a given condition or pathology.

Examples of conditions which may be treated using the compounds or compositions disclosed herein include, but are not limited to, cardiovascular disease, inflammation of airway epithelium, disorders of alveolar tissue, bladder incontinence, impaired hearing due to diseases of the cochlea, endothelial lesions, diabetic retinopathy and diabetic neuropathy, ischemia of the central nervous system and spinal cord, dental tissue disorders including periodontal disease, kidney diseases, failures of bone marrow transplantation, wounds, erectile dysfunction, urinary bladder incontinence, neuropathic pain, subchronic and chronic inflammation, cancer and failures of bone marrow and stem cell transplantation, conditions which arise during transplantation of cells and tissues or during medical procedures such as surgery; as well as conditions caused by an excess of reactive oxygen species and/or free radicals and/or nitric oxide.

### METHODS

### Peptide Synthesis of Cyclic Compounds

A preferred general peptide synthesis procedure is described below. However, more detailed descriptions of solid phase peptide syntheses are found in WO 01/62775 hereby incorporated by reference in its entirety.

Peptides are synthesized batchwise in a polyethylene vessel equipped with a polypropylene filter for filtration using 9-fluorenylmethyloxycarbonyl (Fmoc) as N-α-amino protecting group and suitable common protection groups for side-chain functionalities. All couplings are continued for at least 2 hours. All cyclizations are performed according to the synthesis as described in WO 01/62775. The acylations are checked by the ninhydrin test performed at 80 °C as earlier described. After completed synthesis the peptide-resin is washed with DMF (3x 15 ml, 1 min each), DCM (3x 15 ml, 1 min each), diethyl ether (3x 15 ml, 1 min each) and dried in vacuo. The peptide is then cleaved from the resin as described above and freeze-dried. After purification using preparative HPLC as described above, the peptide product are collected and the identity of the peptide was confirmed by ES-MS.

### Surface Plasmon Resonance (SPR)

SPR is a spectroscopic method to determine binding amplitude and kinetics in real time (Salamon et al., "Surface Plasmon Resonance Spectroscopy as a Tool for Investigating the Biochemical and Biophysical Properties of Membrane Protein Systems. II: Applications to Biological Systems," Biochim. Biophys. Acta 1331(2):131-152 (1997); Duffy et al., "Functional Demonstration of Connexin-protein Binding Using Surface Plasmon Resonance," Cell Adhes. Commun. 8(4-6):225-229 (2001); Lang et al., "Surface Plasmon Resonance as a Method to Study the Kinetics and Amplitude of Protein-protein Binding," in Practical Methods in Cardiovascular Research 936-947 (Stefan Dhein, Friedrich Wilhelm Mohr & Mario Delmar eds., 2005)). Recombinant Cx43CT is covalently bound to a carboxylmethyl dextran matrix (Salamon et al., "Surface Plasmon Resonance Spectroscopy as a Tool for Investigating the Biochemical and Biophysical Properties of Membrane Protein Systems. II: Applications to Biological Systems," Biochim. Biophys. Acta 1331(2):131-152 (1997)). Specific peptide compounds are presented and, when feasible, dissociation constants (KD) are calculated from the time course of binding and unbinding of the ligand, using a 1:1 (Langmuir) association and dissociation kinetic model (Biacore software package). In both phases (association and dissociation), the first 5-8 seconds of recording are not included in the fit, to avoid artifacts resulting from peptide distribution within the flow cells (Lang et al., "Surface Plasmon Resonance as a Method to Study the Kinetics and Amplitude of Protein-protein Binding," in Practical Methods in Cardiovascular Research 936-947 (Stefan Dhein, Friedrich Wilhelm Mohr & Mario Delmar eds., 2005), which is hereby incorporated by reference in its entirety). In these experiments recombinant rat Cx43CT was covalently bound to a carboxylmethyl dextran matrix and used as ligand.

### Electrophysiological Analysis.

Patch clamp experiments were conducted in N2a cells transiently transfected with Cx43. In all cases, the dual-whole-cell voltage clamp technique was used to record gap junction currents. Specifics for cell culture, transfection and recordings are described in Shibayama et al. (Identification of a novel peptide that interferes with the chemical regulation of connexin 43. Circ Res 2006 98:1365-72). Octanol superfusion was initiated 5 minutes after patch break and continued for 10 minutes. Concentration of octanol was 1.5 mM in all experiments. Peptides were diluted in the internal pipette solution to a final concentration of 100 µM. A total of 20 experiments were carried out where octanol-induced uncoupling was tested in the absence of peptides. These experiments were averaged, and data used as control for comparison with those series where a given peptide was assessed.

### Quantitative analysis of Cx43CT-compound interactions.

The screening method described in Phase I allows identification of compounds that do not bind to Cx43CT. A preliminary ranking order may be assigned to those compounds that bind to the target. In the second phase, more detailed quantitative parameters may be established to define the Cx43CT-compound interaction.

Specific Experiments:
1) Determination of Cx43CT-compound dissociation constant. The binding kinetics of each compound to Cx43CT is assessed by SPR. Various concentrations of ligate are used and the rate of association and dissociation is fitted with a Langmuir 1:1 kinetic model (Biacore). This model assumes first-rate order kinetics of binding. Proper fitting will yield an estimation of KD. In some cases, the interaction of the compound to the Cx43CT may depart from a uni-uni model, in which case KD values are not generated.
2) Concentration-dependence of binding using cross-linking reagents. This system allows the establishment of a quantitative parameter for compound-Cx43Ct association even if the binding kinetics are not suitable for KD determinations. Cross-linking is tested for a constant concentration of Cx43CT and sequential dilutions of the compound. The ratio of bound versus unbound densities is plotted as a function of compound concentration. The peptide concentration corresponding to half-maximum binding is defined as the apparent EC50.
3) Identification of compound-induced Cx43CT resonance shifts by nuclear magnetic resonance (NMR). Previous studies have identified those amino acids in Cx43CT whose position in space is affected by RXP4 and by RXPE. The resonance map for Cx43CT is repeated with candidate compounds.

### EXPERIMENTAL EXAMPLES

The invention will now be further illustrated with reference to the following examples. It will be appreciated that what follows is by way of example only and that modifications to detail may be made while still falling within the scope of the invention.

### EXAMPLES

### Example 1 - Peptide Synthesis of Cyclic Compounds

The following compounds of Table 1 were synthesized and representative examples of them tested in the following examples.

**Table 1: Sequence and MW of synthesized compounds**

| **ZP #** | **Sequence** | **MW average (g/mol)** |
|---|---|---|
| 2769 | Cyclo(RRPPYQ) | 797.92 |
| 2771 | Cyclo(RRPPYRQ) | 954.11 |
| 2778 | Cyclo(RRPPYN) | 783.89 |
| 2782 | Cyclo(RRPPWN) | 806.93 |

### Example 2 - Surface Plasmon Resonance (SPR)

Examples of the cyclic peptide compounds of the present invention were presented to the bound ligand, and the amplitude of the response was subtracted from that obtained from a control chamber with no ligand. No change in angle of resonance was taken as an indication of absence of binding. The amplitude of the change in angle of resonance (expressed as "response units") is a function of the mass of the analyte and therefore, for comparison, response units were calibrated by the molecular weight of the tested compound. Change in angle of incidence of resonance (in "resonance units") was plotted against time after onset of both linear and cyclic peptide exposure.

**Table 2: SPR data on cyclic and linear peptides at different concentrations.**

| **Peptide** | **Mw** | **Conc** | **Resonance** | **(ru*100)/Mw** |
|---|---|---|---|---|
| **ZP #** | **g/mol** | **mM** | **Units (ru)** | |
| 2769 | 797,92 | 1,000 | 196,2 | 24,59 |
| | 797,92 | 0,500 | 101,9 | 12,77 |
| | 797,92 | 0,250 | 55,8 | 6,99 |
| | 797,92 | 0,125 | 29,6 | 3,71 |
| | 797,92 | 0,063 | 18,6 | 2,33 |
| 2771 | 953,53 | 1,000 | 1253,0 | 131,41 |
| | 953,53 | 0,500 | 896,0 | 93,97 |
| | 953,53 | 0,250 | 574,0 | 60,20 |
| | 953,53 | 0,125 | 323,6 | 33,94 |
| | 953,53 | 0,063 | 192,4 | 20,18 |
| | 953,53 | 0,031 | 95,8 | 10,05 |
| | 953,53 | 0,016 | 58,1 | 6,09 |
| | 953,53 | 0,008 | 27,6 | 2,89 |

SPR allows for assessment of ligand-analyte binding in real time, and was used for characterization of two of the cyclic peptide compounds of the present invention. Cx43CT was covalently bound to the matrix of a sensor chip. Table 2 shows the change in angle of incidence of resonance was recorded, thus indicating direct binding of cyclic peptide compounds to Cx43CT. In Figures 1A and 1B, the change in angle of incidence of resonance (in "resonance units") was plotted against time after onset of cyclic peptide exposure. Different concentrations was applied represented by different colors. Both peptides ZP2769 (figure 1A) and ZP2771 (figure 1 B) show significant binding to the Cx43CT, although ZP2769 seems to be less effective.

### Example 3 - Electrophysiological Analysis.

Junctional conductance was measured in N2a cell pairs expressing Cx43. Patch pipettes were filled with an internal solution containing the specific peptide under study, and the time course and extent of octanol-induced uncoupling was compared to that observed in the absence of the peptide. Figures 2-5 show average measurements of junctional conductance and percent of coupled cells recorded at various times after the onset of octanol superfusion. Red symbols and line correspond to data obtained when the cyclized peptides ZP2769, ZP2771, ZP2778, ZP2782 were dissolved in the internal pipette solution, and black symbols correspond to the average time course of octanol-induced uncoupling in control conditions. The data show that, in the presence of the cyclized peptides, the progression of octanol-induced uncoupling was significantly delayed and the minimum value of average Gj recorded at the end of the 10-minute octanol exposure was significantly different from that obtained in the absence of the peptides (p<<0.05). Moreover, for the peptides ZP2769 (Fig. 2) and ZP2771 (Fig. 3), all cells remained coupled ten minutes after the onset of octanol superfusion. For ZP2778 (Fig. 4) 60% of the cells remained coupled while 20% remained coupled for ZP2782 (Fig. 5).

## Claims

1. A compound which is a cyclic peptide or a cyclic peptidomimetic compound represented by Formula I
cyclo [Arg¹-Arg²-Pro³-pro⁴- Tyr⁵-(Arg⁶)ₙ-X⁷]
wherein
n is 0 or 1
X₇ is selected from Asn or a Glx group represented by the formula: 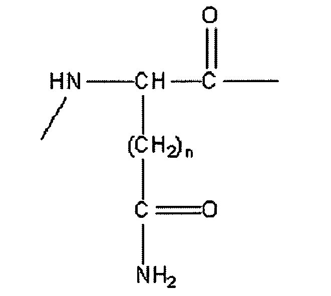 and n is 0, 2 or 6 and
X₇ is linked to Arg1 via a peptide bond to cyclise the peptide or peptidomimetic compound;
or a compound which differs from Formula I at one, two or three of the following positions whereby, if different from Formula I:
the residue at position Arg¹, Arg² and/or Arg⁶ is independently replaced by Lys, His or a Lysine mimetic group;
the residue at position Pro³ and/or Pro⁴ is independently replaced by a Proline mimetic selected from azetidine, hydroxyproline, morphoplino-3-carboxylic acid or an N-substituted amino acid such as Sarcosine, N-cyclohexylglycine or N-phenylglycine or 1-amino-cyclopentane carboxylic acid (Ac5c) or 1-amino-cyclohexane carboxylic acid (Ac6c);
the residue at position Tyr⁵ is replaced by Trp, naphthylalanine , Phe, Met, Val, Ile or Leu;
or a retro analogue or a pharmaceutically acceptable salt thereof.

2. The compound according to claim 1, wherein the compound comprises one, two or three changes in which the residue at position Arg¹, Arg² and/or Arg⁶ is independently replaced by Lys, His or a Lysine mimetic group and/or the residue at position Tyr⁵ is replaced by Trp, naphthylalanine , Phe, Met, Val, Ile or Leu.

3. The compound according to claim 1 or claim 2, wherein the compound comprises a change at position Arg¹ in which the residue is replaced by Lys, His or a Lysine mimetic group.

4. The compound according to any one of claims 1 to 3, wherein the compound comprises a change at position Arg² in which the residue is replaced by Lys, His or a Lysine mimetic group.

5. The compound according to any one of claims 1 to 4, wherein the compound comprises a change at position Arg⁶ in which the residue is replaced by Lys, His or a Lysine mimetic group.

6. The compound according to any one of claims 1 to 5, wherein the compound comprises a change at position Tyr⁵ in which the residue is replaced by Trp, naphthylalanine , Phe, Met, Val, Ile or Leu.

7. The compound of any one of the preceding claims, which is Cyclo(RRPPYQ), Cyclo(RRPPYRQ), Cyclo(RRPPYN), Cyclo(RRPPWN), or a retro analogue or a pharmaceutically acceptable salt thereof.

8. A pharmaceutical composition comprising one or more compounds according to any one of claims 1 to 7 and a pharmaceutical carrier.

9. The pharmaceutical composition according to claim 8, wherein the composition is orally administrable.

10. A method for modulating gap junctional communication in a population of cells comprising administering an effective amount of a peptide according to any one of claims 1 to 7 to a population of cells thereby modulating gap junctional communication between the cells.

11. A compound according to any one of claims 1 to 7 for use in method of medical treatment.

12. The compound according to claim 11, wherein the medical treatment is selected from the group consisting of a cardiovascular disease, inflammation of airway epithelium, a disorder of alveolar tissue, impaired hearing, an endothelial lesion, diabetic retinopathy, diabetic neuropathy, ischemia of the central nervous system, ischemia of the spinal cord, a dental tissue disorder, osteroporosis, kidney disease, failure of bone marrow transplantation, wound, erectile dysfunction, urinary bladder incontinence, neuropathic pain, subchronic and chronic inflammation, cancer, transplantation failure; dermal disorders such as psoriasis and conditions caused by an excess of reactive oxygen species and/or free radicals and/or nitric oxide.

13. Use of a compound of any of claims 1 to 7 in the manufacture of a medicament for the treatment of a pathological condition involving impaired gap junctional communication.

14. The use according to claim 13, wherein the patient is a human being.

15. The use according to claim 13 or claim 14, wherein the pathological condition is selected from the group consisting of a cardiovascular disease, inflammation of airway epithelium, a disorder of alveolar tissue, impaired hearing, an endothelial lesion, diabetic retinopathy, diabetic neuropathy, ischemia of the central nervous system, ischemia of the spinal cord, a dental tissue disorder, osteroporosis, kidney disease, failure of bone marrow transplantation, wound, erectile dysfunction, urinary bladder incontinence, neuropathic pain, subchronic and chronic inflammation, cancer, transplantation failure; dermal disorders such as psoriasis and conditions caused by an excess of reactive oxygen species and/or free radicals and/or nitric oxide.
